# EUROPÄISCHE PATENTSCHRIFT

(11) **EP 0 775 695 B1**
(45) Veröffentlichungstag und Bekanntmachung des Hinweises auf die Patenterteilung: **07.02.2001**
(21) Anmeldenummer: 96118085.8
(22) Anmeldetag: 12.11.1996
(51) Int. Cl.: C07D 209/48

(54) **Verfahren zur Herstellung von N-Cyclohexylthiophthalimid**
Process for the preparation of N-cyclohexylthiophthalimide
Procédé pour la préparation de N-cyclohexylthiophtalimide

(30) Priorität: 24.11.1995 DE 19543863
(43) Veröffentlichungstag der Anmeldung: 28.05.1997
(73) Patentinhaber: BAYER AG, 51368 Leverkusen (DE)
(72) Erfinder: Sicheneder, Adolf, Dr., 25551 Hohenlockstedt (DE); Nolte, Wilfried, Dr., 51519 Odenthal (DE); Schlesmann, Harro, Dr., 51519 Odenthal (DE); Kleiner, Thomas, Dr., 51519 Odenthal (DE)

(56) Entgegenhaltungen:
- EP-A- 0 047 912
- US-A- 3 579 460
- CHEMICAL ABSTRACTS, vol. 98, no. 13, 28.März 1983 Columbus, Ohio, US; abstract no. 107158h, Seite 589; XP002024424
- CHEMICAL ABSTRACTS, vol. 118, no. 22, 31.Mai 1993 Columbus, Ohio, US; abstract no. 214691e, Seite 84; XP002024423
- CHEMICAL ABSTRACTS, vol. 111, no. 7, 14.August 1989 Columbus, Ohio, US; abstract no. 57537v, Seite 741; XP002024425
- CHEMICAL ABSTRACTS, vol. 117, no. 19, 9.November 1992 Columbus, Ohio, US; abstract no. 191681q, Seite 784; XP002024426
- CHEMICAL ABSTRACTS, vol. 109, no. 11, 12.September 1988 Columbus, Ohio, US; abstract no. 92777d, Seite 687; XP002024427
- CHEMICAL ABSTRACTS, vol. 70, no. 13, 31.März 1969 Columbus, Ohio, US; abstract no. 57337q, Seite 315; XP002024428

## Beschreibung

Die Erfindung betrifft ein Verfahren zur Herstellung von N-Cyclohexylthio-phthalimid durch Umsetzung von Dicyclohexyldisulfid, Phthalimid und Chlor in Gegenwart einer Base.

Die erfindungsgemäß hergestellte Verbindung wird zur Verzögerung der Anvulkanisation von natürlichen und/oder synthetischen Kautschuken verwendet.

N-Cyclohexylthio-phthalimid kann durch Umsetzung von Phthalimid mit Cyclohexylsulfenylchlorid hergestellt werden. Cyclohexylsulfenylchlorid kann man durch Chlorierung von Cyclohexylmercaptan oder Dicyclohexyldisulfid erzeugen. Vorzugsweise geht man bei der Herstellung von Cyclohexylsulfenylchlorids so vor, daß man Cyclohexylmercaptan in Wasser suspensiert, mit Wasserstoffperoxid oxidiert, das entstandene Dicyclohexyldisulfid mit einem organischen Lösungsmittel, vorzugsweise einen bei Raumtemperatur flüssigen, gegebenenfalls chlorierten Kohlenwasserstoff wie Toluol oder Hexan, extrahiert und das gelöste Disulfid mit Chlor zum Cyclohexylsulfenylchlorid chloriert. Die Reaktionen entsprechen also dem Schema:

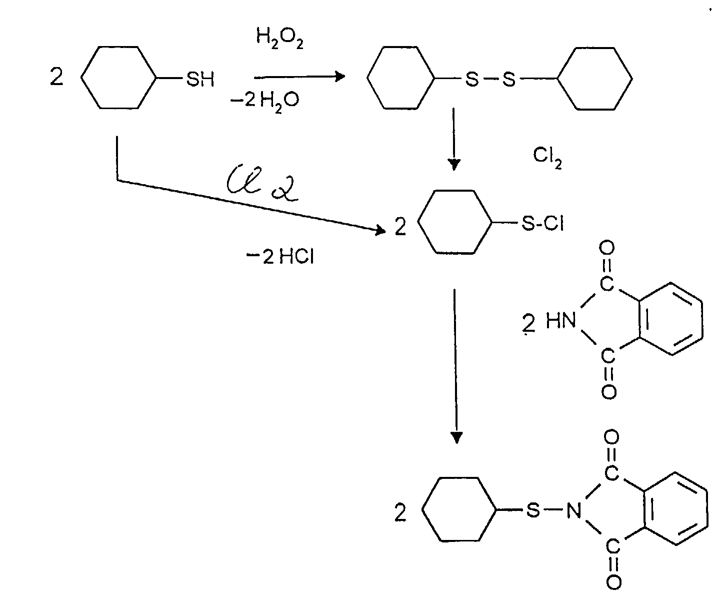

Bei der letzten Stufe des Verfahrens setzt man gemäß US-PS 3 579 460 in Dimethylformamid gelöstes Phthalimid mit in Pentan gelöstem Cyclohexylsulfenylchlorid in Gegenwart einer äquimolaren Menge tertiären Amins um. Die Fällung des Endprodukts mit einer großen Menge Wasser führt zu einem großen Aufwand für die Rückgewinnung von Amin und Lösungsmittel, für die Aufarbeitung der Mutterlauge und für die Trocknung.

Die EP-A 47 912 beschreibt ein Verfahren zur Herstellung von N-(Cyclohexylthio)-phthalimid durch Umsetzung von Phthalimid mit Cyclohexylsulfenylchlorid in Gegenwart einer Base, welches dadurch gekennzeichnet ist, daß als Base ein Alkali- oder Erdalkalihydroxyl eingesetzt wird. Die Reproduzierbarkeit der dort angegebenen Äusbeuten kann jedoch nicht immer befriedigen.

Aus Chem. Abs., 1983, 98:107158h ist bekannt, N-(Cyclohexylthio)-phthalimid herzustellen, indem man Dicyclohexyldisulfid und Chlor in eine Suspension von Kaliumphthalimid bei 40 °C leitet, das Reaktionsgemisch 120 bis 240 Minuten bei 20 °C reagieren läßt und danach das entstehende Kaliumchlorid abfiltriert.

Die angegebenen Ausbeuten an Cyclohexylthiophthalimid sind jedoch für eine großtechnische Produktion noch nicht befriedigend.

Überraschenderweise wurde nun gefunden, daß die Ausbeuten innerhalb sehr enger Grenzen reproduzierbar sind, wenn man nach dem Schema 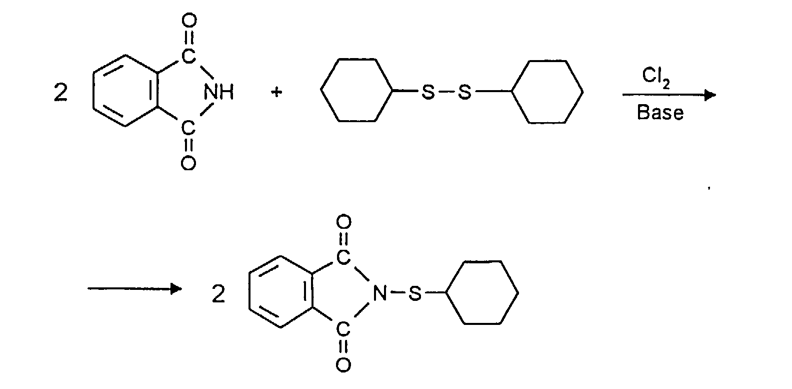 in situ aus Dicyclohexyldisulfid hergestelltes Cyclohexylsulfenylchlorid in Gegenwart einer Base auf in organischem Lösungsmittel suspendiertes Phthalimid einwirken läßt. Hierdurch entfällt die Handhabung der hydrolyseempfindlichen Cyclohexylsulfenylchloridlösung. Dies bewirkt deutliche Verbesserungen im Bereich Umweltschutz und Arbeitssicherheit.

Gegenstand der Erfindung ist also ein Verfahren zur Herstellung von N-Cyclohexylthiophthalimid durch Umsetzung von Dicyclohexyldisulfid in Gegenwart von Chlor und einer Base, dadurch gekennzeichnet, daß man zu einem Gemisch, das aus Toluol und/oder Hexan und/oder Cyclohexan, Phthalimid und Dicyclohexyldisulfid besteht, bei 0 bis 20 °C Chlor einleitet und anschließend diesem Gemisch ohne Isolierung des in situ gebildeten Cyclohexylsulfenylchlorids als Base ein tertiäres Amin oder eine wäßrige Lösung von Alkali- oder Erdalkalihydroxiden zufügt, wobei das Molverhältnis Base/Phthalimid 1:1 bis 1,3:1 beträgt.

Das Molverhältnis Dicyclohexyldisulfid/Phthalimid kann 1:1,9 bis 1:2,2, vorzugsweise 1:2 bis 1:2,1 betragen.

Das Molverhältnis Chlor/Dicyclohexyldisulfid kann 1:1 bis 1,2:1, vorzugsweise 1:1 bis 1,05:1 betragen.

Als Basen sind Alkali- und Erdalkalihydroxide (vorzugsweise als wäßrige Lösungen eingesetzt) und tertiäre Amine bevorzugt. Das Molverhältnis Base/Phthalimid kann innerhalb weiter Grenzen schwanken; es beträgt im allgemeinen 1:1 bis 1,3:1, vorzugsweise 1,05:1 bis 1,1:1.

Bevorzugte Alkali- und Erdalkalihydroxide umfassen Natrium-, Kalium-, Lithium- und Calciumhydroxid. Vorzugsweise wird Natronlauge in Konzentration von 5 bis 40 Gew.-% eingesetzt. Besonders vorteilhaft ist es, wenn die Dichte der eingesetzten Hydroxidlösung bei der gewünschten Reaktionstemperatur im Bereich von 1,0 bis 1,2 g/ml liegt.

Bevorzugte tertiäre Amine entsprechen der Formel

NR¹R²R³

worin R¹ - R³ unabhängig voneinander C₂-C₆-Alkyl, C₆-C₁₂-Cycloalkyl, C₇-C₁₂-Aralkyl, C₆-C₁₂-Aryl bedeuten, wobei zwei dieser Reste zusammen auch eine gegebenenfalls durch ein Sauerstoffatom unterbrochene C₄-C₇-Alkylengruppe bilden können.

Bevorzugte tertiäre Amine umfassen beispielsweise Triethylamin, Dimethylbenzylamin und N-Ethylmorpholin.

Als Lösungsmittel werden in das erfindungsgemäße Verfahren Toluol und/oder Hexan und/oder Cyclohexan eingesetzt.

Die Mengen Lösungsmittel betragen in der Regel 600 bis 1.500 Gew.-%, bezogen auf Phthalimid.

### Beispiele

Die in den nachfolgenden Beispielen genannten Teile sind Gewichtsteile; Prozentangaben beziehen sich auf das Gewicht.

### Beispiel 1

In einem Rührgefäß werden vorgelegt:
- 300: Teile Toluol
- 40: Teile Phthalimid
- 31: Teile Dicyclohexyldisulfid.

Das Gemisch wird auf 5°C abgekühlt. Dann leitet man unter Rühren zunächst
- 9,8: Teile Chlor
ein. Anschließend pumpt man
- 38,2: Teile Dimethylbenzylamin
ein und rührt noch 15 Minuten nach. Die resultierende Lösung wäscht man mit
- 80: Teilen Salzsäure (5 %ig)
und trennt dann die organische Phase ab.

Man destilliert im Vakuum 200 Teile Toluol ab und fällt das Produkt durch Zugabe von Hexan. Man filtriert das feste Produkt ab, wäscht mit 30 Teilen Hexan und trocknet das Produkt im Vakuum.
- Ausbeute:: 68 Teile N-(Cyclohexylthio)-phthalimid = 97 % der Theorie, bezogen auf das eingesetzte Dicyclohexyldisulfid.

Der Gehalt an reiner Substanz beträgt 98 %.

### Beispiel 2

In einem Rührgefäß werden vorgelegt:
- 300: Teile Toluol
- 41: Teile Phthalimid
- 30: Teile Dicyclohexyldisulfid.

Das Gemisch wird auf 5°C abgekühlt. Dann leitet man
- 9,5: Teile Chlor ein. Anschließend werden
- 115: Teile Natronlauge (10 %ig)
zugepumpt und 30 Minuten nachgerührt. Nach Phasentrennung wird das Produkt aus der organischen Phase wie unter Beispiel 1 isoliert.
- Ausbeute:: 66 Teile N-(Cyclohexylthio)-phthalimid = 97 % der Theorie, bezogen auf das eingesetzte Dicyclohexyldisulfid.

Der Gehalt an reiner Substanz beträgt 99 %.

## Patentansprüche

1. Verfahren zur Herstellung von N-Cyclohexylthiophthalimid durch Umsetzung von Dicyclohexyldisulfid in Gegenwart von Chlor und einer Base, dadurch gekennzeichnet, daß man zu einem Gemisch, das aus Toluol und/oder Hexan und/oder Cyclohexan, Phthalimid und Dicyclohexyldisulfid besteht, bei 0 bis 20 °C Chlor einleitet und anschließend diesem Gemisch ohne Isolierung des in situ gebildeten Cyclohexylsulfenylchlorids als Base ein tertiäres Amin oder eine wäßrige Lösung von Alkali- oder Erdalkalihydroxiden zufügt, wobei das Molverhältnis Base/Phthalimid 1:1 bis 1,3:1 beträgt.

## Claims

1. A process for preparing N-cyclohexylthiophthalimide by reacting dicyclohexyl disulfide in the presence of chlorine and a base, characterised in that chlorine is passed into a mixture consisting of toluene and/or hexane and/or cyclohexane, phthalimide and dicyclohexyl disulfide at 0 to 20°C and then, as a base, a tertiary amine or an aqueous solution of alkali metal or alkaline earth metal hydroxides is added to this mixture without isolating the cyclohexyl sulfenyl chloride formed in situ, wherein the molar ratio of base/phthalimide is 1:1 to 1.3:1.

## Revendications

1. Procédé pour la préparation de N-cyclohexylthiophtalimide par réaction de disulfure de dicyclohexyle en présence de chlore et d'une base, caractérisé en ce que l'on introduit dans un mélange qui est constitué de toluène et/ou d'hexane et/ou de cyclohexane, de phtalimide et de disulfure de dicyclohexyle du chlore à de 0 à 20°C et on ajoute ensuite à ce mélange sans isoler le chlorure de cyclohexylsulfényle formé in situ une amine tertiaire ou une solution aqueuse d'hydroxydes alcalins ou alcalino-terreux en tant que base, le rapport molaire base/phtalimide étant compris entre 1:1 et 1,3:1.
